# EUROPEAN PATENT APPLICATION

(11) **EP 3 587 421 A1**
(43) Date of publication of application: **01.01.2020**
(21) Application number: 18180779.3
(22) Date of filing: 29.06.2018
(51) Int. Cl.: C07D 487/04, A61K 31/4985, A61P 35/00

(54) **CRYSTALLINE FORMS OF (S)-4-(8-AMINO-3-(1-(BUT-2-YNOYL)PYRROLIDIN-2-YL)IMIDAZO[1,5-ALPHA]PYRAZIN-1-YL-N-(PYRIDIN-2-YL)BENZAMIDE**

(71) Applicant: Sandoz AG, 4056 Basel (CH)
(72) Inventor: The designation of the inventor has not yet been filed
(74) Representative: Kluschanzoff, Harald

(57) **Abstract**

The present invention relates to crystalline acalabrutinib *L*-pyroglutamate and crystalline acalbrutinib L-malate and to processes for their preparation. Furthermore, the invention relates to a pharmaceutical composition comprising one of said crystalline forms, preferably in a predetermined and/or effective amount and at least one pharmaceutically acceptable excipient. The pharmaceutical composition of the present invention can be used as a medicament, in particular for the treatment of cancers such as mantle cell lymphoma and/or chronic lymphocytic leukaemia.

## Description

### FIELD OF THE INVENTION

The present invention relates to crystalline acalabrutinib *L*-pyroglutamate and crystalline acalbrutinib *L*-malate and to processes for their preparation. Furthermore, the invention relates to a pharmaceutical composition comprising one of said crystalline forms, preferably in a predetermined and/or effective amount and at least one pharmaceutically acceptable excipient. The pharmaceutical composition of the present invention can be used as a medicament, in particular for the treatment of cancers such as mantle cell lymphoma and/or chronic lymphocytic leukaemia.

### BACKGROUND OF THE INVENTION

(*S*)-4-(8-Amino-3-(1-(but-2-ynoyl)pyrrolidin-2-yl)imidazo[1,5-α]pyrazine-1-yl)-*N*-(pyridine-2-yl)benazmide or 4-[8-amino-3-[(2*S*)-1-(1-oxo-2-butyn-1-yl)-2-pyrrolidinyl]imidazo[1,5-α]pyrazin-1-yl]-*N*-2-pyridinylbenzamide also known as acalabrutinib is represented by the following chemical structure according to Formula (I)

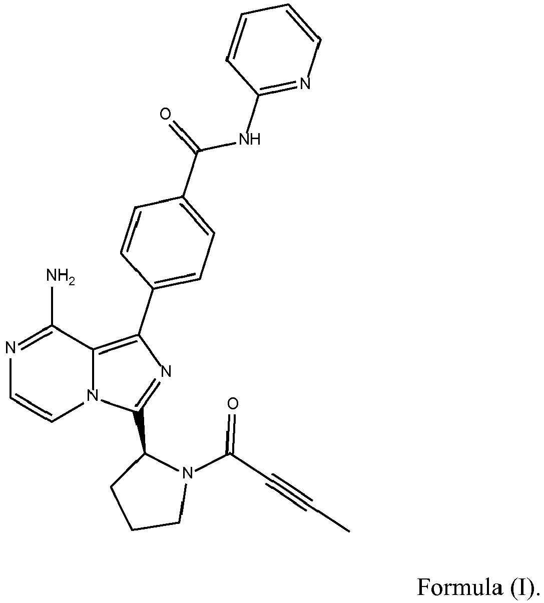

Acalabrutinib is an inhibitor of Bruton tyrosine kinase (BTK), whereat acalabrutinib and its active metabolite, ACP-5862, form a covalent bond with a cysteine residue in the BTK active site, leading to inhibition of enzymatic activity. Currently approved in the US for the second line treatment of patients with mantle cell lymphoma (MCL), the product (Calquence®) is on the market as capsules for oral use, whereat each capsule contains 100 mg acalabrutinib free base as active ingredient.

(*S*)-4-(8-Amino-3-(1-(but-2-ynoyl)pyrrolidin-2-yl)imidazo[1,5-α]pyrazine-1-yl)-*N*-(pyridine-2-yl)benazmide free base (acalabrutinib) is disclosed in WO 2013/010868 A1 (Example 6 therein).

WO 2017/002095 A1 discloses various solid forms of acalabrutinib and also pharmaceutically acceptable salts thereof including crystalline forms of the fumarate, maleate, phosphate, *L-*tartrate, citrate, gentisate, oxalate and sulfate salt of acalabrutinib.

WO 2018/064797 A1 discloses crystalline forms of acalabrutinib free base.

Different solid forms of an active pharmaceutical ingredient often possess different properties. Differences in the physicochemical properties of solid state forms can be important for the improvement of pharmaceutical compositions, for example, pharmaceutical formulations with improved dissolution profile or with improved stability or shelf-life can become accessible due to an improved solid state form of an active pharmaceutical ingredient. Also processing or handling of the active pharmaceutical ingredient during the formulation process may be improved. New physical forms of an active pharmaceutical ingredient can thus have desirable processing properties. They can be easier to handle, better suited for storage, and/or allow for better purification, compared to previously known solid state forms.

Acid addition salts and co-crystals for example are a well-established means for improving solubility and dissolution rate of a drug substance. Acalabrutinib exhibits pH-dependent solubility across the physiological pH range. While it demonstrates high solubility under acidic conditions up to pH 4, its solubility decreases dramatically at pH values above 4. In particular, for pharmaceutical compositions, which are not intended to release the drug substance in the acidic environment of the stomach, acalabrutinib acid addition salts can increase the acidic microenvironment and consequently may improve solubility and dissolution rate of acalabrutinib.

However, a satisfactory solid state form of a drug substance must also be technically feasible and suitable for full-scale production and its solid state properties must be maintained batchwise as well as over time. Physicochemical properties which have to be considered for a solid form intended to be used for an oral dosage form are for example physical and chemical stability e.g. against moisture and/or temperature, melting point, hygroscopicity, crystal habit, crystal hardness, compactibility and flowability.

The salts described in WO 2017/002095 A1 possess unfavorable solid state properties in this regard though. For example, they are unstable hydrates, which tend to lose their crystal water at dry conditions and absorb significant amounts of water upon moisture contact. In addition, they are characterized by rather low and sometimes diffuse melting points. Furthermore, they are solvates or contain significant amounts of residual solvent and/or are characterized by needle shaped morphology. All in all the physciochemical properties of the various acalabrutinib salts provided in WO 2017/002095 A1 suggest that they are not the first choice to be used for pharmaceutical processing and storage of an oral dosage form and indeed, the commercial product (Calquence®) contains the free base according to the FDA label.

The objective of the present invention is therefore to provide improved solid forms of acalabrutinib, in particular improved salts and/or co-crystals of acalabrutinib which are physically and/or chemically stable against moisture and/or temperature stress. In particular, the invention relates to improved solid forms of acalabrutinib, in particular improved salts and/or co-crystals of acalabrutinib which exhibit high melting points and low hygroscopicity.

### SUMMARY OF THE INVENTION

The present invention solves one or more of the above mentioned problems by providing crystalline acalabrutinib *L*-pyroglutamate and crystalline acalabrutinib *L*-malate. It was surprisingly found that both forms are physically stable over the whole humidity range from 0 to 90% relative humidity e.g. they preserve their crystal structures and hardly interact with water vapour. In addition, both forms possess significant higher melting points compared to the acalabrutinib salts of the prior art. Hence, crystalline acalabrutinib *L*-pyroglutamate and crystalline acalabrutinib *L*-malate of the present invention possess an unexpected combination of advantageous properties which render them the preferred pyhsical forms of acalbrutinib, in particular the preferred salts and/or co-crystals of acalabrutinib for the preparation of a pharmaceutical composition, in particular an oral dosage form.

### Abbreviations

- PXRD: powder X-ray diffractogram
- FTIR: Fourier transform infrared
- ATR: attenuated total reflection
- DSC: differential scanning calorimetry
- TGA: thermogravimetric analysis
- GMS: gravimetric moisture sorption
- mp: melting point
- decomp.: decomposition
- RH: relative humidity

### Definitions

As used herein the term "room temperature" refers to a temperature in the range of from 20 to 30 °C.

The term "acalabrutinib" as used herein refers to (*S*)-4-(8-Amino-3-(1-(but-2-ynoyl)pyrrolidin-2-yl)imidazo[1,5-α]pyrazine-1-yl)-*N*-(pyridine-2-yl)benazmide or 4-[8-amino-3-[(2*S*)-1-(1-oxo-2-butyn-1-yl)-2-pyrrolidinyl]imidazo[1,5-α]pyrazin-1-yl]-*N*-2-pyridinylbenzamide according to the chemical structure illustrated in Formula (I) herein above. The compound possesses a chiral center, however in the course of the present invention the term encompasses a single enantiomer, namely the (*S)*-enantiomer.

The term "*L*-pyroglutamic acid" as used herein is also known under the name "pidolic acid" and refers to 5-oxopyrrolidine-2-carboxylic acid or 5-oxo-*L*-proline according to the chemical structure illustrated in Formula (a) below

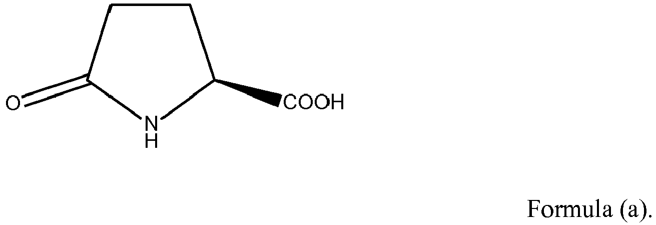

Pyroglutamic acid possesses a chiral center, however in the course of the present invention the term encompasses a single enantiomer, namely the (*L*)-enantiomer.

The term "acalbrutinib *L*-pyroglutamate" as used herein refers to a salt, a co-crystal or a mixture of a salt and a co-crystal of acalabrutinib and *L*-pyroglutamic acid according to the chemical structure illustrated in Formula (IIa) herein below.

The term "*L*-malic acid" as used herein refers to 2-hydroxybutanedioic acid or 2-hydroxysuccinic acid according to the chemical structure illustrated in Formula (b) below

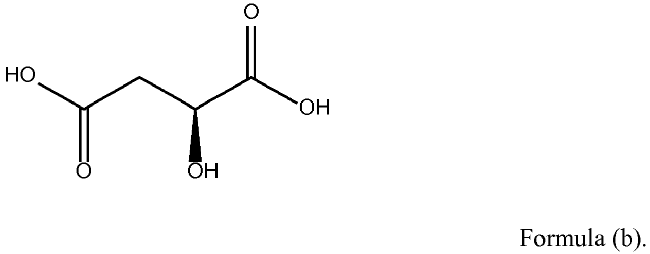

Malic acid possesses a chiral center, however in the course of the present invention the term encompasses a single enantiomer, namely the (*L*)-enantiomer.

The term "acalbrutinib *L*-malate" as used herein refers to a salt, a co-crystal or a mixture of a salt and a co-crystal of acalabrutinib and *L*-malic acid according to the chemical structure illustrated in Formula (IIb) herein below.

As used herein the term "salt" refers to crystalline materials composed of two or more different molecular and/or ionic compounds in the same crystal lattice that are associated by ionic bonds.

The term "co-crystal" as used herein refers to crystalline materials composed of two or more different molecular and/or ionic compounds in the same crystal lattice that are associated by nonionic and noncovalent bonds, wherein at least two of the individual molecular and/or ionic compounds are solids at room temperature.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 0-90% relative humidity, preferably 20-80% relative humidity, more preferably 40-60% relative humidity and most preferably 50% relative humidity.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 6.6° 2-Theta for example can appear between 6.4° and 6.8° 2-Theta, preferably between 6.5 and 6.7° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "essentially the same" with reference to Fourier transform infrared spectroscopy means that variabilities in peak positions and relative intensities of the peaks are to be taken into account. For example, a typical precision of the wavenumber values is in the range of ± 2 cm⁻¹. Thus, a peak at 3318 cm⁻¹ for example can appear in the range of from 3316 to 3320 cm⁻¹ on most infrared spectrometers under standard conditions. Differences in relative intensities are typically smaller compared to X-ray diffraction. However, one skilled in the art will appreciate that small differences in peak intensities due to degree of crystallinity, sample preparation and other factors can also occur in infrared spectroscopy. Relative peak intensities should therefore be taken as qualitative measure only.

The terms "physical form", "solid form" or "solid state form" aas used interchangeably herein refer to any crystalline and/or amorphous phase of a compound. Crystalline phases include anhydrous/non-solvated forms of a compound and their polymorphs, hydrates and solvates of a compound and their polymorphs, salts and co-crystals of a compound and any mixtures thereof.

The terms "anhydrous" or "anhydrate" as used herein refer to a crystalline solid where no water is cooperated in or accommodated by the crystal structure. Anhydrous forms may still contain residual water, which is not part of the crystal structure but may be adsorbed on the surface or absorbed in disordered regions of the crystal.

The term "non-solvated" as used herein, refers to a crystalline solid where no organic solvent is cooperated in or accommodated by the crystal structure. Non-solvated forms may still contain residual organic solvents, which are not part of the crystal structure but may be adsorbed on the surface or absorbed in disordered regions of the crystal.

Crystalline acalabrutinibe *L*-pyroglutamate or crystalline acalabrutinib *L*-malate may be referred to herein as being characterized by a powder X-ray diffractogram or an FTIR spectrum "as shown in" a figure. The person skilled in the art understands that factors such as variations in instrument type, response and variations in sample directionality, sample concentration, sample purity, sample history and sample preparation may lead to variations, for example relating to the exact reflection and peak positions and their intensities. However, a comparison of the graphical data in the figures herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid from said solution.

A "predetermined amount" as used herein with regard to crystalline acalabrutinibe *L-*pyroglutamate or crystalline acalabrutinib *L*-malate refers to the initial amount of crystalline acalabrutinibe *L*-pyroglutamate or crystalline acalabrutinib *L*-malate used for the preparation of a pharmaceutical composition having a desired dosage strength of acalabrutinib.

The term "effective amount" as used herein with regard to crystalline acalabrutinibe *L-*pyroglutamate or crystalline acalabrutinib *L*-malate encompasses an amount of crystalline acalabrutinibe *L*-pyroglutamate or crystalline acalabrutinib *L*-malate, which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient.

The terms "filler" or "diluent" as used herein refer to substances that are used to dilute the active pharmaceutical ingredient prior to delivery. Diluents and fillers can also serve as stabilizers.

The terms "disintegrant" or "disintegrating agent" as used herein refers to substances which, upon addition to a solid pharmaceutical composition, facilitate its break-up or disintegration after administration and permits the release of the active pharmaceutical ingredient as efficiently as possible to allow for its rapid dissolution.

The term "lubricant" as used herein refers to substances which are added to a powder blend to prevent the compacted powder mass from sticking to the equipment during tableting or encapsulation process. They aid the ejection of the tablet from the dies and can improve powder flow.

### BRIEF DESCRIPTION OF THE FIGURES

**Figure 1****:** illustrates a representative PXRD of crystalline acalabrutinib *L*-pyroglutamate of the present invention. The x-axis shows the scattering angle in °2-Theta, the y-axis shows the intensity of the scattered X-ray beam in counts of detected photons.
**Figure 2****:** illustrates a representative FTIR spectrum of crystalline acalabrutinib *L-*pyroglutamate of the present invention. The x-axis shows the wavenumbers in cm⁻¹, the y-axis shows the relative intensity in percent transmittance.
**Figure 3****:** illustrates a representative DSC curve of crystalline acalabrutinib *L*-pyroglutamate of the present invention. The x-axis shows the temperature in degree Celsius (°C), the y-axis shows the heat flow rate in Watt per gram (W/g) with endothermic peaks going up.
**Figure 4****:** illustrates a representative TGA curve of crystalline acalabrutinib *L*-pyroglutamate of the present invention. The x-axis shows the temperature in degree Celsius (°C), the y-axis shows the mass (loss) of the sample in weight percent (weight%).
**Figure 5****:** illustrates a representative PXRD of crystalline acalabrutinib *L*-malate of the present invention. The x-axis shows the scattering angle in °2-Theta, the y-axis shows the intensity of the scattered X-ray beam in counts of detected photons.
**Figure 6****:** illustrates a representative FTIR spectrum of crystalline acalabrutinib *L*-malate of the present invention. The x-axis shows the wavenumbers in cm⁻¹, the y-axis shows the relative intensity in percent transmittance.
**Figure 7****:** illustrates a representative DSC curve of crystalline acalabrutinib *L*-malate of the present invention. The x-axis shows the temperature in degree Celsius (°C), the y-axis shows the heat flow rate in Watt per gram (W/g) with endothermic peaks going up.
**Figure 8****:** illustrates a representative TGA curve of crystalline acalabrutinib *L*-malate of the present invention. The x-axis shows the temperature in degree Celsius (°C), the y-axis shows the mass (loss) of the sample in weight percent (weight%).

### DETAILED DESCRIPTION OF THE INVENTION

The present invention provides crystalline acalabrutinib *L*-pyroglutamate and crystalline acalabrutinib *L*-malate, two forms of acalabrutinib which exhibit significantly improved physicochemical properties compared to the prior art salts of WO 2017/002095 A1. In particular the crystalline forms of the present invention are more stable against moisture and temperature stress e.g. they preserve their crystal structure when subjected to moisture and/or temperature stress, are only slightly hygroscopic and exhibit high melting points.

### Crystalline acalabrutinib L-pyroglutamate

In a first aspect, the present invention relates to crystalline acalabrutinib *L*-pyroglutamate.

Preferably, the invention relates to crystalline acalabrutinib *L*-pyroglutamate represented by the chemical structure according to Formula (IIa) 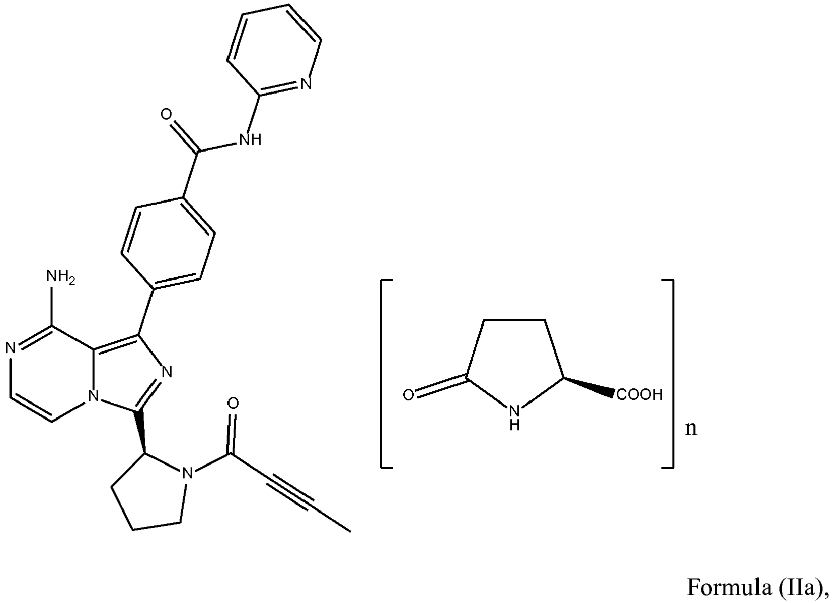 wherein n is in the range of from 0.8 to 1.2, preferably of from 0.9 to 1.1, more preferably of from 0.95 to 1.05 and most preferably n is 1.0. In a particular embodiment, the invention relates to crystalline acalabrutinib *mono*-*L*-pyroglutamate. Crystalline acalabrutinib *L*-pyroglutamate according to the present invention may be present as salt, co-crystal or salt/co-crystal mixture.

Crystalline acalabrutinib *L*-pyroglutamate of the present invention may be characterized by analytical methods well known in the field of the pharmaceutical industry for characterizing crystalline solids. Such methods comprise but are not limited to PXRD, FTIR DSC, TGA and GMS. Crystalline acalabrutinib *L*-pyroglutamate of the present invention may be characterized by one of the aforementioned analytical methods or by combining two or more of them. In particular, crystalline acalabrutinib *L*-pyroglutamate of the present invention may be characterized by any one of the following embodiments or by combining two or more of the following embodiments.

In one embodiment the present invention relates to crystalline acalabrutinib *L*-pyroglutamate which can be characterized by having a PXRD comprising reflections at 2-Theta angles of:
(6.6 ± 0.2)°, (19.4 ± 0.2)° and (22.2 ± 0.2)°; or
(6.6 ± 0.2)°, (12.4 ± 0.2)°, (19.4 ± 0.2)° and (22.2 ± 0.2)°; or
(6.6 ± 0.2)°, (10.5 ± 0.2)°, (12.4 ± 0.2)°, (19.4 ± 0.2)° and (22.2 ± 0.2)°; or
(6.6 ± 0.2)°, (8.9 ± 0.2)°, (10.5 ± 0.2)°, (12.4 ± 0.2)°, (19.4 ± 0.2)° and (22.2 ± 0.2)°; or
(6.6 ± 0.2)°, (8.9 ± 0.2)°, (10.5 ± 0.2)°, (12.4 ± 0.2)°, (13.1 ± 0.2)°, (19.4 ± 0.2)° and (22.2 ± 0.2)°; or
(6.6 ± 0.2)°, (8.9 ± 0.2)°, (10.5 ± 0.2)°, (12.4 ± 0.2)°, (13.1 ± 0.2)°, (14.0 ± 0.2)°, (19.4 ± 0.2)° and (22.2 ± 0.2)°; or
(6.6 ± 0.2)°, (8.9 ± 0.2)°, (10.5 ± 0.2)°, (12.4 ± 0.2)°, (13.1 ± 0.2)°, (14.0 ± 0.2)°, (19.4 ± 0.2)°, (19.8 ± 0.2)° and (22.2 ± 0.2)°; or
(6.6 ± 0.2)°, (8.9 ± 0.2)°, (10.5 ± 0.2)°, (12.4 ± 0.2)°, (13.1 ± 0.2)°, (14.0 ± 0.2)°, (19.4 ± 0.2)°, (19.8 ± 0.2)°, (22.2 ± 0.2)° and (22.6 ± 0.2)°,
when measured at a temperature in the range of from 20 to 30 °C with Cu-Kalpha_{1,2} radiation having a wavelength of 0.15419 nm.

In another embodiment the present invention relates to crystalline acalabrutinib *L-*pyroglutamate which can be characterized by having a PXRD comprising reflections at 2-Theta angles of:
(6.6 ± 0.1)°, (19.4 ± 0.1)° and (22.2 ± 0.1)°; or
(6.6 ± 0.1)°, (12.4 ± 0.1)°, (19.4 ± 0.1)° and (22.2 ± 0.1)°; or
(6.6 ± 0.1)°, (10.5 ± 0.1)°, (12.4 ± 0.1)°, (19.4 ± 0.1)° and (22.2 ± 0.1)°; or
(6.6 ± 0.1)°, (8.9 ± 0.1)°, (10.5 ± 0.1)°, (12.4 ± 0.1)°, (19.4 ± 0.1)° and (22.2 ± 0.1)°; or
(6.6 ± 0.1)°, (8.9 ± 0.1)°, (10.5 ± 0.1)°, (12.4 ± 0.1)°, (13.1 ± 0.1)°, (19.4 ± 0.1)° and (22.2 ± 0.1)°; or
(6.6 ± 0.1)°, (8.9 ± 0.1)°, (10.5 ± 0.1)°, (12.4 ± 0.1)°, (13.1 ± 0.1)°, (14.0 ± 0.1)°, (19.4 ± 0.1)° and (22.2 ± 0.1)°; or
(6.6 ± 0.1)°, (8.9 ± 0.1)°, (10.5 ± 0.1)°, (12.4 ± 0.1)°, (13.1 ± 0.1)°, (14.0 ± 0.1)°, (19.4 ± 0.1)°, (19.8 ± 0.1)° and (22.2 ± 0.1)°; or
(6.6 ± 0.1)°, (8.9 ± 0.1)°, (10.5 ± 0.1)°, (12.4 ± 0.1)°, (13.1 ± 0.1)°, (14.0 ± 0.1)°, (19.4 ± 0.1)°, (19.8 ± 0.1)°, (22.2 ± 0.1)° and (22.6 ± 0.1)°,
when measured at a temperature in the range of from 20 to 30 °C with Cu-Kalpha_{1,2} radiation having a wavelength of 0.15419 nm.

In yet another embodiment the present invention relates to crystalline acalabrutinib *L-*pyroglutamate which can be characterized by having a PXRD comprising no reflections in the range of from 2.0 to 6.3° 2-Theta, preferably of from 2.0 to 6.4° 2-Theta, when measured at a temperature in the range of from 20 to 30 °C with Cu-Kalpha_{1,2} radiation having a wavelength of 0.15419 nm.

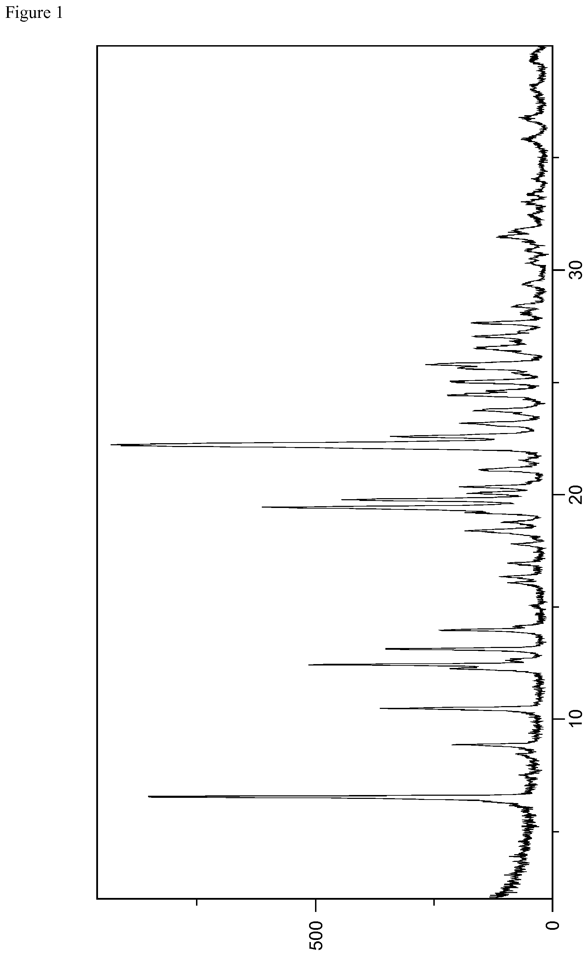

In still another embodiment the present invention relates to crystalline acalabrutinib *L-*pyroglutamate characterized by having a PXRD essentially the same as shown in Figure 1 of the present invention, when measured at a temperature in the range of from 20 to 30 °C with Cu-Kalpha_{1,2} radiation having a wavelength of 0.15419 nm.

In a further embodiment, the present invention relates to crystalline acalabrutinib *L-*pyroglutamate characterized by having an FTIR spectrum comprising peaks at wavenumbers of:
(3318 ± 2) cm⁻¹, (2225 ± 2) cm⁻¹ and (1625 ± 2) cm⁻¹ or;
(3318 ± 2) cm⁻¹, (2225 ± 2) cm⁻¹, (1625 ± 2) cm⁻¹ and (1304 ± 2) cm⁻¹; or
(3318 ± 2) cm⁻¹, (2225 ± 2) cm⁻¹, (1625 ± 2) cm⁻¹, (1525 ± 2) cm⁻¹ and (1304 ± 2) cm⁻¹; or
(3318 ± 2) cm⁻¹, (2225 ± 2) cm⁻¹, (1625 ± 2) cm⁻¹, (1525 ± 2) cm⁻¹, (1436 ± 2) cm⁻¹ and (1304 ± 2) cm⁻¹; or
(3318 ± 2) cm⁻¹, (2225 ± 2) cm⁻¹, (1625 ± 2) cm⁻¹, (1525 ± 2) cm⁻¹, (1436 ± 2) cm⁻¹, (1304 ± 2) cm⁻¹ and (1240 ± 2) cm⁻¹; or
(3318± 2) cm⁻¹, (2225± 2) cm⁻¹, (1625 ± 2) cm⁻¹, (1525 ± 2) cm⁻¹, (1436 ± 2) cm⁻¹, (1304 ± 2) cm⁻¹, (1240 ± 2) cm⁻¹ and (961 ± 2) cm⁻¹; or
(3318 ± 2) cm⁻¹, (2225 ± 2) cm⁻¹, (1625 ± 2) cm⁻¹, (1525 ± 2) cm⁻¹, (1436 ± 2) cm⁻¹, (1304 ± 2) cm⁻¹, (1240 ± 2) cm⁻¹, (961 ± 2) cm⁻¹ and (725 ± 2) cm⁻¹; or
(3318 ± 2) cm⁻¹, (2981 ± 2) cm⁻¹, (2225 ± 2) cm⁻¹, (1625 ± 2) cm⁻¹, (1525 ± 2) cm⁻¹, (1436 ± 2) cm⁻¹, (1304 ± 2) cm⁻¹, (1240 ± 2) cm⁻¹, (961 ± 2) cm⁻¹ and (725 ± 2) cm⁻¹,
when measured at a temperature in the range of from 20 to 30 °C with a diamond ATR cell.

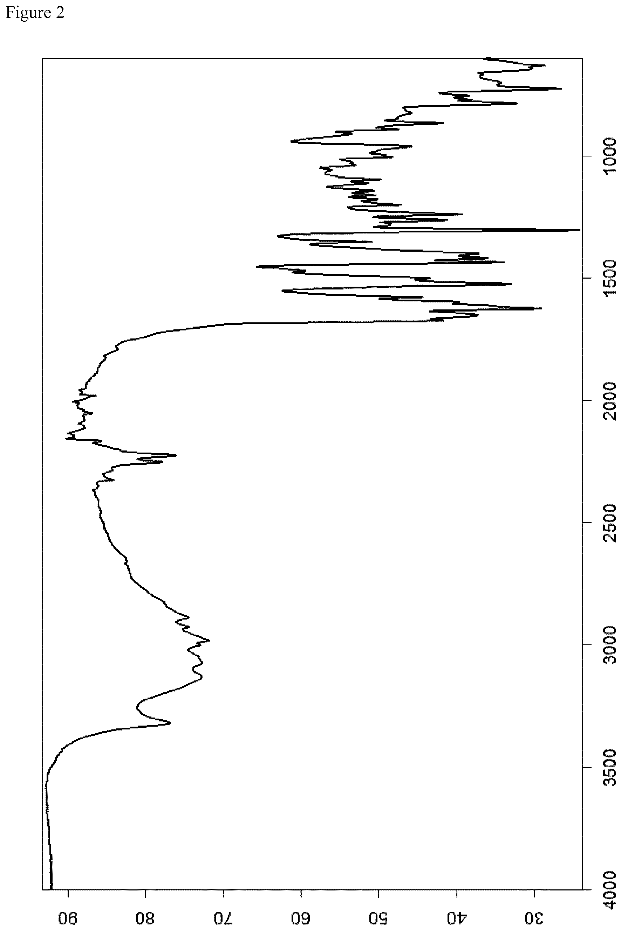

In yet another embodiment, the present invention relates to crystalline acalabrutinib *L-*pyroglutamate characterized by having an FTIR spectrum essentially the same as shown in Figure 2 of the present invention, when measured at a temperature in the range of from 20 to 30 °C with a diamond ATR cell.

In one embodiment the present invention relates to crystalline acalabrutinib *L*-pyroglutamate characterized by having a DSC curve comprising an endothermic peak with an onset temperature in the range of (228 ± 5) °C, preferably of (228 ± 2) °C, more preferably of (228 ± 1) °C, when measured at a heating rate of 10 K/min.

In another embodiment the present invention relates to crystalline acalabrutinib *L-*pyroglutamate characterized by having a DSC curve comprising an endothermic peak with a peak maximum temperature in the range of (230 ± 5) °C, preferably of (230 ± 2) °C, more preferably of (230 ± 1) °C, when measured at a heating rate of 10 K/min.

In yet another embodiment the invention relates to crystalline acalabrutinib *L*-pyroglutamate characterized by having a TGA curve showing a mass loss of not more than 2.0 weight%, preferably of not more than 1.0 weight%, such as not more than 0.8 weight% based on the weight of crystalline acalabrutinib L-pyroglutamate, when measured up to a temperature of 190 °C at a heating rate of 10 K/min. Most preferably, crystalline acalabrutinib L-pyroglutamate of the present invention is a non-solvated form.

In a further embodiment the invention relates to crystalline acalabrutinib *L*-pyroglutamate characterized by a water uptake of not more than 2.0 weight%, preferably of not more than 1.5 weight%, more preferably of not more than 1.0 weight% based on the weight of crystalline acalabrutinib *L*-pyroglutamate, when measured with gravimetric moisture sorption at a relative humidity in the range of from 0 to 80% at a temperature of (25.0 ± 0.1) °C.

In yet another embodiment the invention relates to crystalline acalabrutinib *L*-pyroglutamate characterized by a water uptake of not more than 2.0 weight%, such as not more than 1.9 weight% based on the weight of crystalline acalabrutinib *L*-pyroglutamate, when measured with gravimetric moisture sorption at a relative humidity in the range of from 0 to 90% at a temperature of (25.0 ± 0.1) °C. Preferably, crystalline acalabrutinib *L*-pyroglutamate of the present invention is an anhydrous form.

Another aspect of the invention concerns a process for the preparation of crystalline acalabrutinib *L*-pyroglutamate of the present invention comprising the steps of:
(a) reacting acalabrutinib with *L*-pyroglutamic acid in the presence of a suitable solvent;
(b) crystallizing acalabrutinib *L*-pyroglutamic acid;
(c) separating at least a part of the crystals obtained in (b) from their mother liquor;
(d) optionally washing the crystals obtained in (c);
(e) optionally drying the crystals obtained in (b), (c) or (d).

Acalabrutinib can be prepared according to the teaching of WO 2013/010868 A1, in particular according to the process disclosed in Example 6 therein. *L*-pyroglutamic acid is commercially available. Suitable solvents which can be used for the reaction of acalabrutinib with *L-*pyroglutamic acid are selected from the group consisting of alcohols such as methanol, ethanol and isopropanol or cyclic ethers such as tetrahydrofuran or any mixtures thereof. A suitable solvent mixture is for example methanol/tetrahydrofuran in a 1:1 volume ratio. Most preferably, ethanol e.g. absolute ethanol or aqueous ethanol (96 volume%) is used for the reaction. The reaction may for example be performed by concomitantly or separately dissolving acalabrutinib and *L*-pyroglutamic acid in one of the solvent/solvent mixtures described above. If the components are dissolved separately e.g. in separate vessels the respective solutions are combined subsequently. The respective solutions may be prepared at increased temperature e.g. at the boiling temperature of the solvent used or at room temperature. The temperature during the acid base reaction is not critical. The reaction may be performed at increased temperature e.g. at the boiling temperature of the solvent used or at room temperature.

Once the reaction is complete crystallization of acalabrutinib *L*-pyroglutamate may be initiated by any conventional method known in the art such as cooling, solvent evaporation, stirring, seeding, scratching with a glass rod and/or antisolvent addition. Preferably, the reaction mixture is stirred at room temperature until crystallization occurs. The preferred acalabrutinib *L-*pyroglutamate concentration during crystallization is in the range of from 50 to 100 g/L, preferably of from 60 to 70 g/L. Once acalabrutinib *L*-pyroglutamate crystallized, the obtained suspension may be further stirred at room temperature or cooled to a temperature in the range of from 0 to 10 °C in order to increase the yield.

Optionally, in the next step, at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

In a further optional step, the isolated crystals may be washed with a suitable solvent. Suitable solvents which can be used for the washing step are selected from the group consisting of alcohols such as methanol, ethanol and isopropanol or cyclic ethers such as tetrahydrofuran or any mixtures thereof. A suitable solvent mixture is for example methanol/tetrahydrofuran in a 1:1 volume ratio. Most preferably, ethanol e.g. absolute ethanol or aqueous ethanol (96 volume%) is used for the washing step. In a particular preferred embodiment the solvent/solvent mixture used in steps (a) and (d) is the same.

Optionally, the crystals obtained from any one of steps (b), (c) or (d) may be subsequently dried, wherein drying is performed at a temperature in the range of from about 20 to 150 °C, preferably of from about 20 to 100 °C, more preferably of from about 20 to 60 °C. Drying may be performed at ambient pressure or under vacuum e.g. in the range of from about 1 to 50 mbar, for example in the range of from about 5 to 30 mbar. Typically, drying is performed for a period in the range of from about 6 to 72 hours, preferably of from about 10 to 48 hours, more preferably of from about 12 to 24 hours.

### Crystalline acalabrutinib L-malate

In a second aspect, the present invention relates to crystalline acalabrutinib *L*-malate.

Preferably, the invention relates to crystalline acalabrutinib *L*-malate represented by the chemical structure according to Formula (IIb) 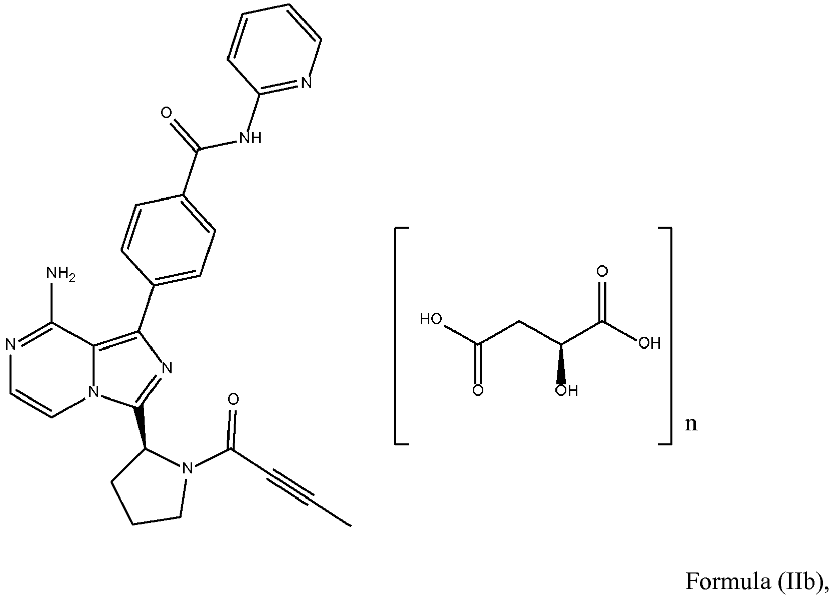 wherein n is in the range of from 0.8 to 1.2, preferably of from 0.9 to 1.1, more preferably of from 0.95 to 1.05 and most preferably n is 1.0. In a particular embodiment, the invention relates to crystalline acalabrutinib *mono-L*-malate. Crystalline acalabrutinib *L*-malate according to the present invention may be present as salt, co-crystal or salt/co-crystal mixture.

Crystalline acalabrutinib *L*-malate of the present invention may be characterized by analytical methods well known in the field of the pharmaceutical industry for characterizing crystalline solids. Such methods comprise but are not limited to PXRD, FTIR DSC, TGA and GMS.

Crystalline acalabrutinib *L*-malate of the present invention may be characterized by one of the aforementioned analytical methods or by combining two or more of them. In particular, crystalline acalabrutinib *L*-malate of the present invention may be characterized by any one of the following embodiments or by combining two or more of the following embodiments.

In one embodiment the present invention relates to crystalline acalabrutinib *L*-malate which can be characterized by having a PXRD comprising reflections at 2-Theta angles of:
(6.2 ± 0.2)°, (11.9 ± 0.2)° and (12.4 ± 0.2)°; or
(6.2 ± 0.2)°, (8.9 ± 0.2)°, (11.9 ± 0.2)° and (12.4 ± 0.2)°; or
(6.2 ± 0.2)°, (8.9 ± 0.2)°, (11.9 ± 0.2)°, (12.4 ± 0.2)° and (22.9 ± 0.2)°; or
(6.2 ± 0.2)°, (8.9 ± 0.2)°, (11.9 ± 0.2)°, (12.4 ± 0.2)°, (22.9 ± 0.2)° and (23.8 ± 0.2)°; or
(6.2 ± 0.2)°, (8.9 ± 0.2)°, (11.9 ± 0.2)°, (12.4 ± 0.2)°, (16.9 ± 0.2)°, (22.9 ± 0.2)° and (23.8 ± 0.2)°; or
(4.2 ± 0.2)°, (6.2 ± 0.2)°, (8.9 ± 0.2)°, (11.9 ± 0.2)°, (12.4 ± 0.2)°, (16.9 ± 0.2)°, (22.9 ± 0.2)° and (23.8 ± 0.2)°; or
(4.2 ± 0.2)°, (6.2 ± 0.2)°, (8.4 ± 0.2)°, (8.9 ± 0.2)°, (11.9 ± 0.2)°, (12.4 ± 0.2)°, (16.9 ± 0.2)°, (22.9 ± 0.2)° and (23.8 ± 0.2)°; or
(4.2 ± 0.2)°, (6.2 ± 0.2)°, (8.4 ± 0.2)°, (8.9 ± 0.2)°, (11.9 ± 0.2)°, (12.4 ± 0.2)°, (16.9 ± 0.2)°, (20.7 ± 0.2)°, (22.9 ± 0.2)° and (23.8 ± 0.2)°; or
(4.2 ± 0.2)°, (6.2 ± 0.2)°, (8.4 ± 0.2)°, (8.9 ± 0.2)°, (11.9 ± 0.2)°, (12.4 ± 0.2)°, (16.9 ± 0.2)°, (19.6 ± 0.2)°, (20.7 ± 0.2)°, (22.9 ± 0.2)° and (23.8 ± 0.2)°; or
(4.2 ± 0.2)°, (6.2 ± 0.2)°, (8.4 ± 0.2)°, (8.9 ± 0.2)°, (11.9 ± 0.2)°, (12.4 ± 0.2)°, (15.6 ± 0.2)°, (16.9 ± 0.2)°, (19.6 ± 0.2)°, (20.7 ± 0.2)°, (22.9 ± 0.2)° and (23.8 ± 0.2)°;
when measured at a temperature in the range of from 20 to 30 °C with Cu-Kalpha_{1,2} radiation having a wavelength of 0.15419 nm.

In another embodiment, the present invention relates to crystalline acalabrutinib *L*-malate which can be characterized by having a PXRD comprising reflections at 2-Theta angles of:
(6.2 ± 0.1)°, (11.9 ± 0.1)° and (12.4 ± 0.1)°; or
(6.2 ± 0.1)°, (8.9 ± 0.1)°, (11.9 ± 0.1)° and (12.4 ± 0.1)°; or
(6.2 ± 0.1)°, (8.9 ± 0.1)°, (11.9 ± 0.1)°, (12.4 ± 0.1)° and (22.9 ± 0.1)°; or
(6.2 ± 0.1)°, (8.9 ± 0.1)°, (11.9 ± 0.1)°, (12.4 ± 0.1)°, (22.9 ± 0.1)° and (23.8 ± 0.1)°; or
(6.2 ± 0.1)°, (8.9 ± 0.1)°, (11.9 ± 0.1)°, (12.4 ± 0.1)°, (16.9 ± 0.1)°, (22.9 ± 0.1)° and (23.8 ± 0.1)°; or
(4.2 ± 0.1)°, (6.2 ± 0.1)°, (8.9 ± 0.1)°, (11.9 ± 0.1)°, (12.4 ± 0.1)°, (16.9 ± 0.1)°, (22.9 ± 0.1)° and (23.8 ± 0.1)°; or
(4.2 ± 0.1)°, (6.2 ± 0.1)°, (8.4 ± 0.1)°, (8.9 ± 0.1)°, (11.9 ± 0.1)°, (12.4 ± 0.1)°, (16.9 ± 0.1)°, (22.9 ± 0.1)° and (23.8 ± 0.1)°; or
(4.2 ± 0.1)°, (6.2 ± 0.1)°, (8.4 ± 0.1)°, (8.9 ± 0.1)°, (11.9 ± 0.1)°, (12.4 ± 0.1)°, (16.9 ± 0.1)°, (20.7 ± 0.1)°, (22.9 ± 0.1)° and (23.8 ± 0.1)°; or
(4.2 ± 0.1)°, (6.2 ± 0.1)°, (8.4 ± 0.1)°, (8.9 ± 0.1)°, (11.9 ± 0.1)°, (12.4 ± 0.1)°, (16.9 ± 0.1)°, (19.6 ± 0.1)°, (20.7 ± 0.1)°, (22.9 ± 0.1)° and (23.8 ± 0.1)°; or
(4.2 ± 0.1)°, (6.2 ± 0.1)°, (8.4 ± 0.1)°, (8.9 ± 0.1)°, (11.9 ± 0.1)°, (12.4 ± 0.1)°, (15.6 ± 0.1)°, (16.9 ± 0.1)°, (19.6 ± 0.1)°, (20.7 ± 0.1)°, (22.9 ± 0.1)° and (23.8 ± 0.1)°;
when measured at a temperature in the range of from 20 to 30 °C with Cu-Kalpha_{1,2} radiation having a wavelength of 0.15419 nm.

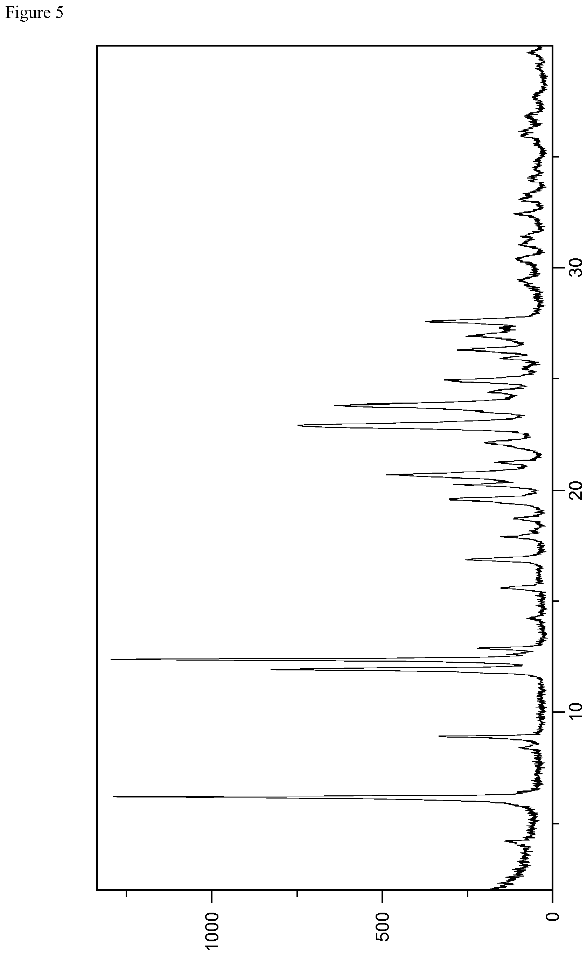

In yet another embodiment, the present invention relates to crystalline acalabrutinib *L*-malate characterized by having a PXRD essentially the same as shown in Figure 5 of the present invention, when measured at a temperature in the range of from 20 to 30 °C with Cu-Kalpha_{1,2} radiation having a wavelength of 0.15419 nm.

In a further embodiment, the present invention relates to crystalline acalabrutinib *L*-malate characterized by having an FTIR spectrum comprising peaks at wavenumbers of:
(3471 ± 2) cm⁻¹, (2223 ± 2) cm⁻¹ and (1626 ± 2) cm⁻¹ or;
(3471 ± 2) cm⁻¹, (2223 ± 2) cm⁻¹, (1626 ± 2) cm⁻¹ and (1302± 2) cm⁻¹; or
(3471 ± 2) cm⁻¹, (2223 ± 2) cm⁻¹, (1677 ± 2) cm⁻¹, (1626 ± 2) cm⁻¹ and (1302 ± 2) cm⁻¹; or
(3471 ± 2) cm⁻¹, (2223 ± 2) cm⁻¹, (1677 ± 2) cm⁻¹, (1626 ± 2) cm⁻¹, (1539 ± 2) cm⁻¹ and (1302 ± 2) cm⁻¹; or
(3471 ± 2) cm⁻¹, (2223 ± 2) cm⁻¹, (1677 ± 2) cm⁻¹, (1626 ± 2) cm⁻¹, (1539 ± 2) cm⁻¹, (1302 ± 2) cm⁻¹ and (1112 ± 2) cm⁻¹; or
(3471 ± 2) cm⁻¹, (2223 ± 2) cm⁻¹, (1677 ± 2) cm⁻¹, (1626 ± 2) cm⁻¹, (1539 ± 2) cm⁻¹, (1302 ± 2) cm⁻¹, (1112 ± 2) cm⁻¹ and (1006 ± 2) cm⁻¹; or
(3471 ± 2) cm⁻¹, (2223 ± 2) cm⁻¹, (1677 ± 2) cm⁻¹, (1626 ± 2) cm⁻¹, (1539 ± 2) cm⁻¹, (1302 ± 2) cm⁻¹, (1112 ± 2) cm⁻¹, (1006 ± 2) cm⁻¹ and (784 ± 2) cm⁻¹; or
(3471 ± 2) cm⁻¹, (2885 ± 2) cm⁻¹, (2223 ± 2) cm⁻¹, (1677 ± 2) cm⁻¹, (1626 ± 2) cm⁻¹, (1539 ± 2) cm⁻¹, (1302 ± 2) cm⁻¹, (1112 ± 2) cm⁻¹, (1006 ± 2) cm⁻¹ and (784 ± 2) cm⁻¹,
when measured at a temperature in the range of from 20 to 30 °C with a diamond ATR cell.

In yet another embodiment, the present invention relates to crystalline acalabrutinib *L*-malate characterized by having an FTIR spectrum essentially the same as shown in Figure 5 of the present invention, when measured at a temperature in the range of from 20 to 30 °C with a diamond ATR cell.

In one embodiment the present invention relates to crystalline acalabrutinib *L*-malate characterized by having a DSC curve comprising an endothermic peak with an onset temperature in the range of (182 ± 2) °C, more preferably of (182 ± 1) °C, when measured at a heating rate of 10 K/min.

In another embodiment the present invention relates to crystalline acalabrutinib *L*-malate characterized by having a DSC curve comprising an endothermic peak with a peak maximum temperature in the range of of (184 ± 2) °C, more preferably of (184 ± 1) °C, when measured at a heating rate of 10 K/min.

In yet another embodiment the invention relates to crystalline acalabrutinib *L*-malate characterized by having a TGA curve showing a mass loss of not more than 2.0 weight%, preferably of not more than 1.0 weight%, such as not more than 0.7 weight% based on the weight of crystalline acalabrutinib *L*-malate, when measured up to a temperature of 180 °C at a heating rate of 10 K/min. Most preferably, crystalline acalabrutinib *L*-malate of the present invention is a non-solvated form.

In a further embodiment the invention relates to crystalline acalabrutinib *L*-malate characterized by a water uptake of not more than 2.0 weight%, preferably of not more than 1.5 weight%, such as not more than 1.2 weight% based on the weight of crystalline acalabrutinib *L*-malate, when measured with gravimetric moisture sorption at a relative humidity in the range of from 0 to 80% at a temperature of (25.0 ± 0.1) °C.

In yet another embodiment the invention relates to crystalline acalabrutinib *L*-malate characterized by a water uptake of not more than 2.0 weight%, such as not more than 1.6 weight% based on the weight of crystalline acalabrutinib *L*-malate, when measured with gravimetric moisture sorption at a relative humidity in the range of from 0 to 90% at a temperature of (25.0 ± 0.1) °C. Preferably, crystalline acalabrutinib *L*-malate of the present invention is an anhydrous form.

Another aspect of the invention concerns a process for the preparation of crystalline acalabrutinib *L*-malate of the present invention comprising the steps of:
(a) reacting acalabrutinib with *L*-malic acid in the presence of a suitable solvent;
(b) crystallizing acalabrutinib *L*-malate;
(c) separating at least a part of the crystals obtained in (b) from their mother liquor;
(d) optionally washing the crystals obtained in (c);
(e) optionally drying the crystals obtained in (b), (c) or (d).

Acalabrutinib can be prepared according to the teaching of WO 2013/010868 A1, in particular according to the process disclosed in Example 6 therein. *L*-malic acid is commercially available. Suitable solvents which can be used for the reaction of acalabrutinib with *L*-malic acid are selected from the group consisting of alcohols such as methanol, ethanol and isopropanol or cyclic ethers such as tetrahydrofuran or any mixtures thereof. A suitable solvent mixture is for example methanol/tetrahydrofuran in a 1:1 volume ratio. Most preferably, ethanol e.g. absolute ethanol or aqueous ethanol (96 volume%) is used for the reaction. The reaction may for example be performed by concomitantly or separately dissolving acalabrutinib and *L*-pyroglutamic acid in one of the solvent/solvent mixtures described above. If the components are dissolved separately e.g. in separate vessels the respective solutions are combined subsequently. Solutions may be prepared at increased temperature e.g. at the boiling temperature of the solvent used or at room temperature. The temperature during the acid base reaction is not critical. The reaction may be performed at increased temperature e.g. at the boiling temperature of the solvent used or at room temperature.

Once the reaction is complete crystallization of acalabrutinib *L*-malate may be initiated by any conventional method known in the art such as cooling, solvent evaporation, stirring, seeding, scratching with a glass rod and/or antisolvent addition. Preferably, the reaction mixture is stirred at room temperature until crystallization occurs. Most preferably, the reaction mixture is in addition seeded with crystalline acalabrutinib *L*-malate of the present invention. Seed crystals can for example be prepared as described in Example 3 of the present invention. The preferred acalabrutinib *L*-malate concentration during crystallization is in the range of from 50 to 100 g/L, preferably of from 60 to 70 g/L. Once acalabrutinib *L*-malate crystallized, the obtained suspension may be further stirred at room temperature or cooled to a temperature in the range of from 0 to 10 °C in order to increase the yield.

Optionally, in the next step, at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

In a further optional step, the isolated crystals may be washed with a suitable solvent. Suitable solvents which can be used for the washing step are selected from the group consisting of alcohols such as methanol, ethanol and isopropanol or cyclic ethers such as tetrahydrofuran or any mixtures thereof. A suitable solvent mixture is for example methanol/tetrahydrofuran in a 1:1 volume ratio. Most preferably, ethanol e.g. absolute ethanol or aqueous ethanol (96 volume%) is used for the reaction. In a particular preferred embodiment the solvent/solvent mixture used in steps (a) and (d) is the same.

Optionally, the crystals obtained from any one of steps (b), (c) or (d) may be subsequently dried, wherein drying is performed at a temperature in the range of from about 20 to 150 °C, preferably of from about 20 to 100 °C, more preferably of from about 20 to 60 °C. Drying may be performed at ambient pressure or under vacuum e.g. in the range of from about 1 to 50 mbar, for example in the range of from about 5 to 30 mbar. Typically, drying is performed for a period in the range of from about 6 to 72 hours, preferably of from about 10 to 48 hours, more preferably of from about 12 to 24 hours.

### Advantages

As already mentioned above crystalline acalabrutinib *L*-pyroglutamate and crystalline acalabrutinib *L*-malate of the present invention possess an unexpected combination of advantageous physiochemical properties, which render them especially suitable physical forms of acalabrutinib to be used in pharmaceutical processing and for safe storage of pharmaceutical drug products containing them.

For example, the melting point of an organic substance is one of the first properties measured, with physical forms having high and well-defined (sharp) melting points being preferred for pharmaceutical purposes over low and diffuse melting forms. DSC is a well-established method to investigate thermal events such as melting and other processes like phase transitions, desolvations/dehydrations and decompositions.

The thermal behavior of various acalabrutinib salts during DSC measurement is described in WO 2017/002095 A1. Table 1 below summarizes these thermal events again and provides a comparison with crystalline acalabrutinib *L*-pyroglutamate and crystalline acalabrutinib L-malate of the present invention.

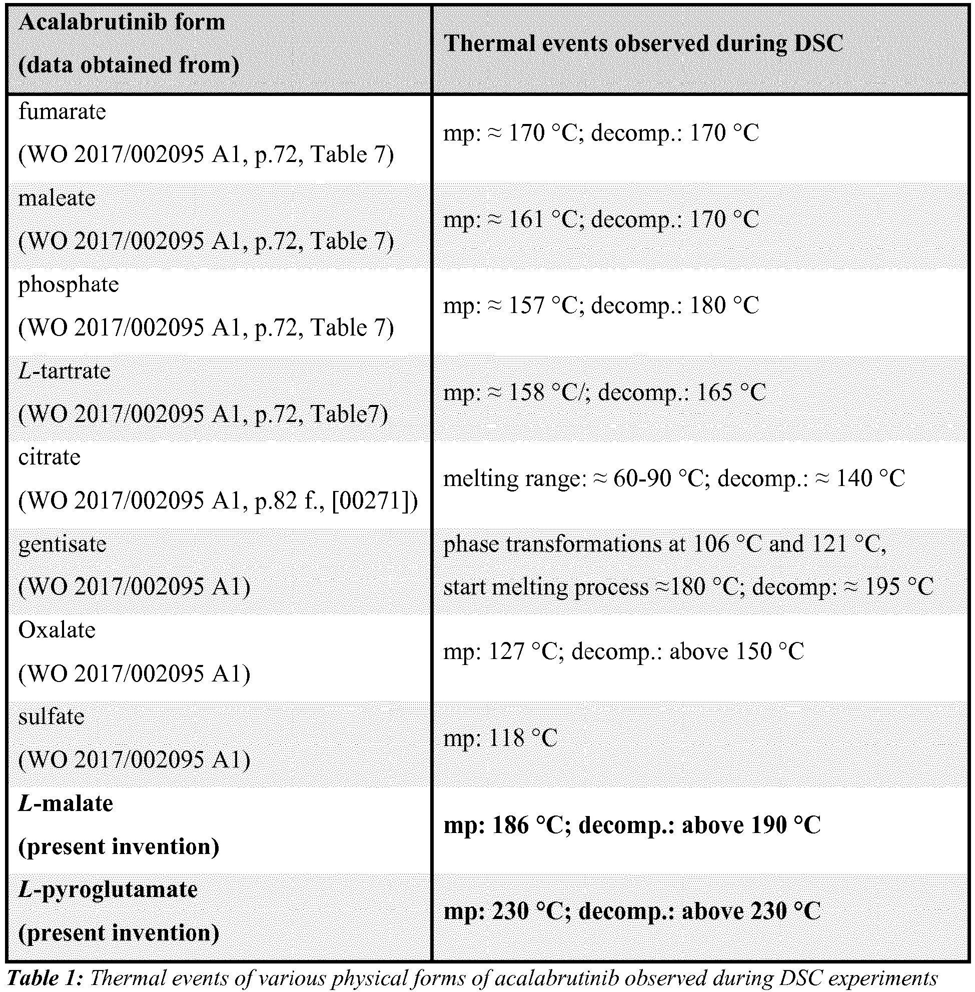

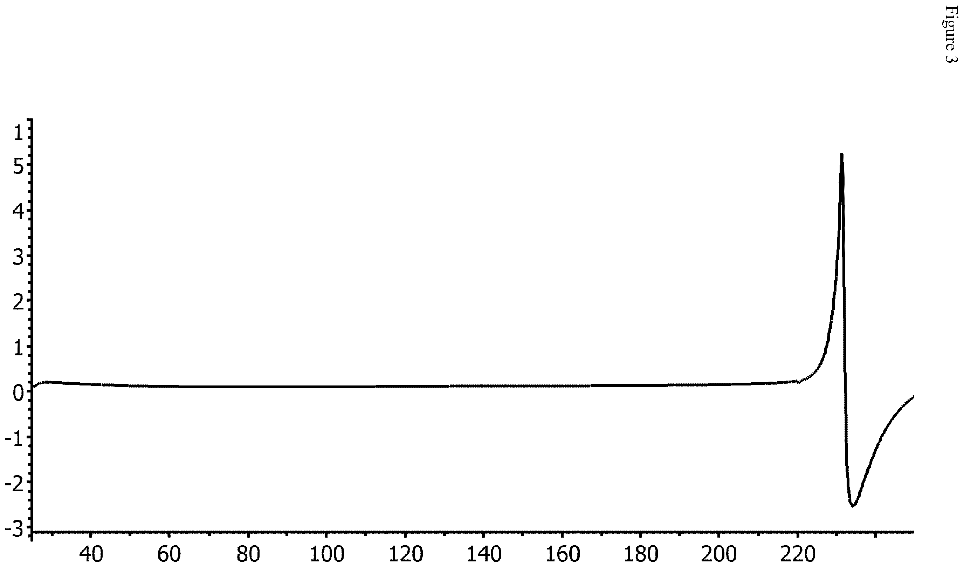

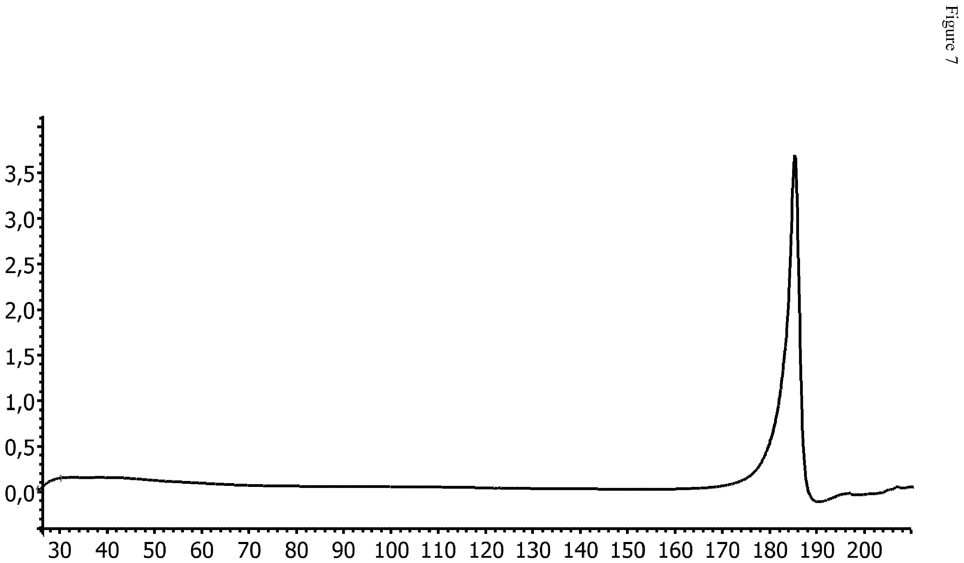

Noteably, the salts of WO 2017/002095 A1 show first thermal events including phase transitions, desolvations/dehydrations and melting/decomposition processes already at relatively low temperatures in the range of from 60 °C to 170 °C. In contrast, crystalline acalabrutinib *L*-pyroglutamate and crystalline acalabrutinib *L*-malate of the present invention show no thermal events in their DSC curves until they sharply melt at relatively high temperatures of 186 °C and 230 °C, respectively (see also DSC curves displayed in Figures 3 and 7 herein). Therefore, both forms of the present invention are significantly more stable against temperature stress according to DSC, with no phase transformations occurring until they melt.

It is noteworthy that the salts disclosed in WO 2017/002095 A1 seem to be hydrates, whereas crystalline acalabrutinib *L*-pyroglutamate and crystalline acalabrutinib *L*-malate of the present invention are anhydrous forms. Particular attention must be paid to the physical stability of hydrates. Hydrates of drug substances can readily be used provided their state of hydration is maintained under all the climatic conditions to which the drug substance and the drug product may be exposed during shelf-life. However, problems will arise, if a hydrate, when exposed to increased temperature or a dry environment, transforms into a lower state of hydration, or to an anhydrous form by loss of crystal water. Table 2 provides an overview of the hydration/dehydration behavior of the hydrated acalabrutinib salts disclosed in WO 2017/002095 A1 and the behavior of the anhydrous crystalline acalabrutinib *L-*pyroglutamate and the anhydrous crystalline acalabrutinib *L*-malate of the present invention during gravimetric moisture sorption.

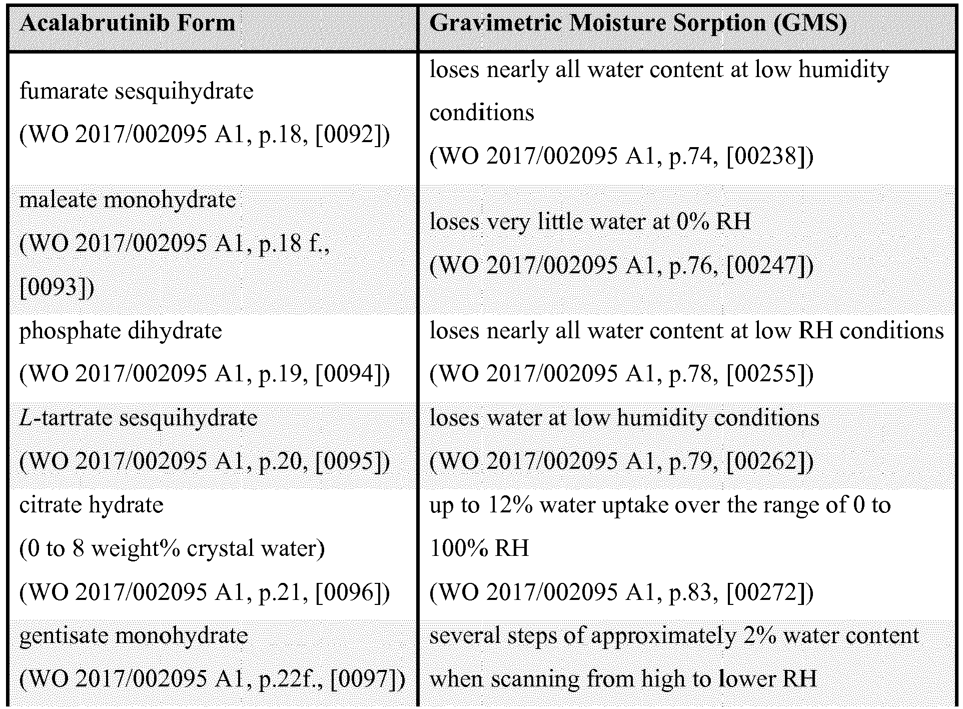

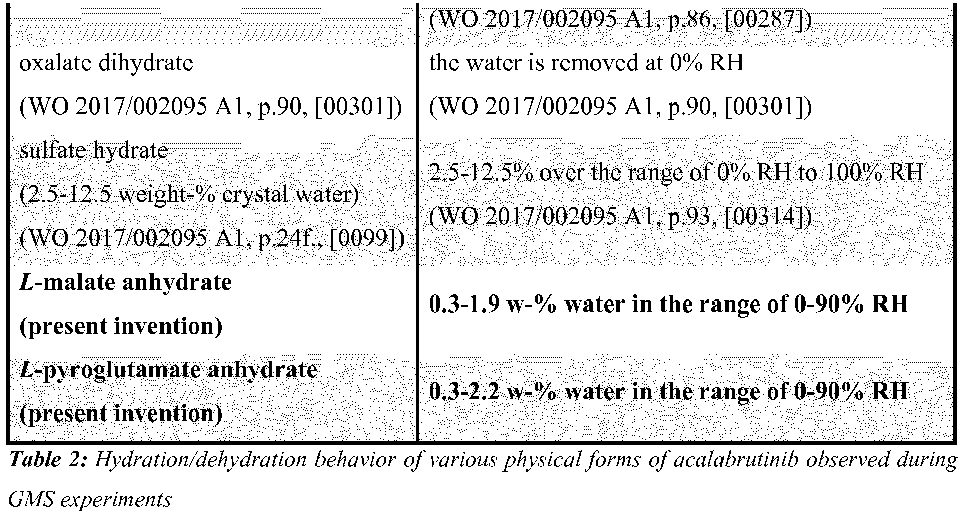

As can be seen from the overview provided in Table 2 above, most of the prior art salts at least partially lose their crystal water at dry condition and some of them significantly take up water at wet conditions. In contrast, the water contents of the anhydrous crystalline acalbrutinib *L-*pyroglutamate and the anhydrous crystalline *L*-malate forms of the present invention remain relatively stable and vary only within a narrow range of less than 2 weight%, which renders pharmaceutical processing and storage of the drug substance and the drug product comprising the same less challenging, since there is no need for precautionary measures.

Hence, in a further aspect the present invention relates to the use of crystalline acalabrutinib *L-*pyroglutamate or crystalline acalabrutinib *L*-malate of the present invention as defined above for the preparation of a pharmaceutical composition.

In another aspect, the present invention relates to a pharmaceutical composition comprising crystalline acalabrutinib *L*-pyroglutamate or crystalline acalabrutinib *L*-malate as defined above, preferably in an effective and/or predetermined amount, and at least one pharmaceutically acceptable excipient. Most preferably, the pharmaceutical composition of the present invention is an oral solid dosage form, such as a tablet or a capsule. Preferably, the pharmaceutical composition of the present invention is a capsule. In one embodiment, the capsule is a hard gelatin capsule. In one embodiment the capsule shell contains gelatin, titanium dioxide, iron oxide and optionally further colorants.

The at least one pharmaceutically acceptable excipient, which is comprised in the pharmaceutical composition of the present invention, is preferably selected from the group consisting of fillers, diluents, lubricants and disintegrants. In one embodiment all of these pharmaceutically acceptable excipients are comprised by the pharmaceutical composition of the present invention.

In a preferred embodiment, the at least one pharmaceutically acceptable excipient is selected from the group consisting of microcrystalline cellulose, e.g. silicified microcrystalline cellulose, partially pregelatinized starch, magnesium stearate and sodium starch glycolate. In a preferred embodiment, all of these pharmaceutically acceptable excipients are comprised by the pharmaceutical composition of the present invention.

Preferably, the present invention relates to a pharmaceutical composition as describe above, wherein the predetermined and/or effective amount of the crystalline acalabrutinib *L-*pyroglutamate or crystalline acalabrutinib *L-*malate is selected from the group consisting of 5 mg, 10 mg, 20 mg, 25 mg, 50 mg and 100 mg calculated as acalabrutinib free base. Most preferably, the invention relates to a pharmaceutical composition as describe above, wherein the predetermined and/or effective amount of crystalline acalabrutinib *L*-pyroglutamate or crystalline acalabrutinib *L*-malate is 100 mg calculated as acalabrutinib free base.

Preferably, the present invention relates to a pharmaceutical composition as described above, wherein the pharmaceutical composition is to be administered twice daily. In one embodiment, the present invention relates to a pharmaceutical composition as described above, wherein the pharmaceutical composition is to be administered every twelve hours.

In a further aspect, the present invention relates to the pharmaceutical composition as described above for use as a medicament.

In yet another aspect, the present invention relates to the pharmaceutical composition as described above for use in the treatment of hyperproliferative disorders. In one embodiment the hyperproliferative disorder is cancer. Preferably, the cancer is selected from the group consisting of chronic lymphocytic leukemia, non-Hodgkin's lymphoma, diffuse large B-cell lymphoma, mantle cell lymphoma, follicular lymphoma and Waldenström's macroglobulinemia. In another embodiment, the cancer is selected from the group consisting of non-Hodgkin's lymphomas (such as diffuse large B-cell lymphoma), acute myeloid leukemia, thymus, brain, lung, squamous cell, skin, eye, retinoblastoma, intraocular melanoma, oral cavity and oropharyngeal, bladder, gastric, stomach, pancreatic, bladder, breast, cervical, head, neck, renal, kidney, liver, ovarian, prostate, colorectal, bone (e.g. metastatic bone), esophageal, testicular, gynecological, thyroid, CNS, PNS, AIDS-related (e.g. lymphoma and Kaposi's sarcoma), viral-induced cancers such as cervical carcinoma (human papillomavirus), B-cell lymphoproliferative disease and nasopharyngeal carcinoma (Eppstein-Barr virus), Kaposi's sarcoma and primary effusion lymphomas (Kaposi's sarcoma herpesvirus), hepatocellular carcinoma (hepatitis B and hepatitis C viruses), and T-cell leukemias (Human T-cell leukemia virus-1), B-cell acute lymphoblastic leukemia, Burkitt's leukemia, juvenile myelomonocytic leukemia, hairy cell leukemia, Hodgkin's disease, multiple myeloma, mast cell leukemia, and mastocytosis. Most preferably, the cancer is selected from mantle cell lymphoma and chronic lymphocytic leukemia.

In another embodiment, the present invention is directed to a method of treating hyperproliferative disorders such as cancer e.g. one or more of the cancer types listed above in particular mantle cell lymphoma and chronic lymphocytic leukemia comprising administering the pharmaceutical composition as described above to a patient in need of such a treatment.

### EXAMPLES

The following non-limiting examples are illustrative for the disclosure and are not to be construed as to be in any way limiting for the scope of the invention.

### Example 1: Preparation of acalabrutinib L-pyroglutamate

Acalabrutinib (500 mg, e.g. prepared according to Examples 6 of WO 2013/010868 A1) was dissolved in ethanol (10 mL) upon heating to 50 °C. To the warm solution *L*-pyroglutamic acid (140 mg) was added. After approximately one minute a suspension was obtained, which was allowed to cool to room temperature and stirred for 16 hours. The obtained crystals were collected by filtration and dried at room temperature under vacuum (5-40 mbar) for 5 hours to obtain crystalline acalabrutinib *L*-pyroglutamate (580 mg, yield: 91% of theory).

### Example 2: Characterization of acalabrutinib L-pyroglutamate

### Powder X-ray diffraction

PXRD was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta. Thus, the diffraction peak of crystalline acalabrutinib *L*-pyroglutamate of the present invention that appears for example at 6.6° 2-Theta can appear in the range of from 6.4 to 6.8° 2-Theta, preferably in the range of from 6.5 to 6.7° 2-Theta on most X-ray diffractometers under standard conditions.

A representative diffractogram of crystalline acalabrutinib *L*-pyroglutamate of the present invention is displayed in Figure 1 herein. The corresponding reflection postitions in the range of 2 to 30 ° 2-Theta are provided in Table 3 below.

**Table 3: PXRD reflections of crystalline acalabrutinib L-pyroglutamate of the present invention is in the range of from 2 to 30° 2-Theta; A typical precision of the 2-Theta values is in the range of ± 0.2° 2-Theta, preferably of ± 0.1° 2-Theta.**

| **Reflection position** **[° 2-Theta]** | **Reflection position** **[° 2-Theta]** | **Reflection position** **[° 2-Theta]** | **Reflection position** **[° 2-Theta]** |
|---|---|---|---|
| 6.6 | 16.1 | 20.4 | 25.6 |
| 7.5 | 16.4 | 21.1 | 25.8 |
| 8.9 | 16.9 | 21.5 | 26.5 |
| 10.5 | 17.8 | 22.2 | 27.1 |
| 12.3 | 18.4 | 22.6 | 27.7 |
| 12.4 | 18.8 | 23.2 | 28.1 |
| 12.6 | 19.2 | 23.8 | 28.4 |
| 13.1 | 19.4 | 24.4 | 29.4 |
| 14.0 | 19.8 | 24.6 | |
| 15.0 | 20.1 | 25.0 | |

### Fourier transform infrared spectroscopy

The FTIR spectrum of acalabrutinib *L*-pyroglutamate was recorded (obtained) on an MKII Golden Gate™ Single Reflection Diamond ATR cell with a Bruker Tensor 27 FTIR spectrometer with 4 cm⁻¹ resolution at a temperature in the range of from 20 to 30 °C. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of ± 2 cm⁻¹. Thus, the infrared peak of crystalline acalabrutinib *L*-pyroglutamate that appears for example at 3318 cm⁻¹ can appear between 3316 and 3320 cm⁻¹ on most infrared spectrometers under standard conditions.

A representative FTIR spectrum of acalabrutinib L-pyroglutamate according to the present invention is displayed in Figure 2 and the corresponding peak list is provided in Table 4 below.

**Table 4: FTIR peak list of crystalline acalabrutinib L-pyroglutamate according to the present invention; a typical precision of the wavenumbers is in the range of ± 2 cm⁻¹.**

| **Wavenumber** **[cm⁻¹]** | **Wavenumber** **[cm⁻¹]** | **Wavenumber** **[cm⁻¹]** | **Wavenumber** **[cm⁻¹]** |
|---|---|---|---|
| 3318 | 1600 | 1263 | 961 |
| 3130 | 1578 | 1240 | 910 |
| 3074 | 1525 | 1200 | 892 |
| 2981 | 1501 | 1179 | 867 |
| 2887 | 1471 | 1161 | 826 |
| 2253 | 1436 | 1143 | 786 |
| 2225 | 1418 | 1111 | 770 |
| 1671 | 1401 | 1098 | 755 |
| 1652 | 1352 | 1037 | 725 |
| 1625 | 1304 | 1005 | 631 |

### Differential scanning calorimetry

DSC was performed on a Mettler Polymer DSC R instrument. Acalabrutinib *L*-pyroglutamate (3.23 mg) was heated in a 40 microliter aluminium pan with a pierced aluminium lid from 25 to 250 °C at a rate of 10 K/min. Nitrogen (purge rate 50 mL/min) was used as purge gas.

The DSC curve of acalabrutinib *L*-pyroglutamate shows an endothermic melting peak with an onset temperature of about 228 °C and a peak maximum temperature of about 230 °C (see also Figure 3 herein). Immediately after melting the sample decomposes, which is indicated by an exothermic peak.

### Thermogravimetric analysis

TGA was performed on a Mettler TGA/DSC 1 instrument. Acalabrutinib *L*-pyroglutamate (8.46 mg) was heated in a 100 microliter aluminum pan closed with an aluminum lid. The lid was automatically pierced at the beginning of the measurement. The sample was heated from 25 to 250 °C at a rate of 10 K/min. Nitrogen (purge rate 50 mL/min) was used as purge gas.

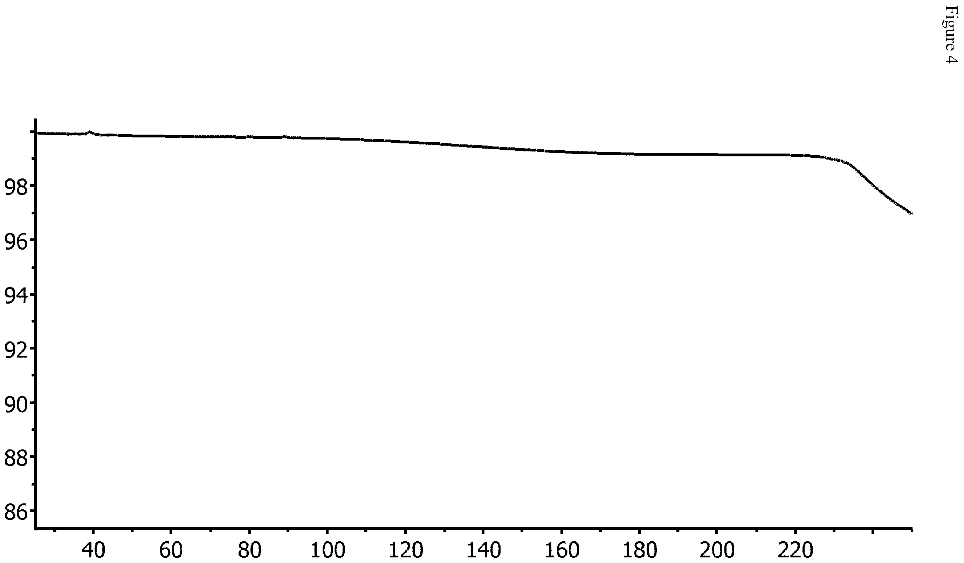

The TGA curve of acalabrutinib *L*-pyroglutamate shows a mass loss of about 0.8 weight% up to a temperature of about 190 °C (see also Figure 4 herein). Hence, it can be concluded that neither water nor organic solvents are part of the crystal structure but the mass loss may rather be due to the release of residual water and/or organic solvent, which is loosely bound on the surface.

### Gravimetric moisture sorption

The moisture sorption isotherm was recorded along with other samples with an SPSx-1µ moisture sorption analyzer (ProUmid, Ulm). In the sorption cycle the relative humidity was increased from 0% to approximately 90% in 5% steps. The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C.

During the sorption cycle crystalline acalabrutinib *L*-pyroglutamate of the present invention shows a mass increase (water uptake) of about 1.0 weight% from 0 to 80% RH and of about 1.9 weight% from 0 to 90% RH.

### Example 3: Preparation of acalabrutinib L-malate

### Preparation of acalabrutinib L-malate seed crystals

A mixture of acalabrutinib (50 mg, e.g. prepared according to Examples 6 of WO 2013/010868 A1), malic acid (14.4 mg) and ethanol (1 mL) was heated to reflux temperature, whereupon a solution was obtained. The solution was allowed to cool to room temperature, which first lead to a gel-like precipitate, before said precipitate dissolved again. The solution was then subsequently stored in the refrigerator at 2-8°C for 16 hours (closed vessel), at room temperature for 6 hours (closed vessel), at 2-8 °C for 4 days (closed vessel), at room temperature for 16 hours (open vessel) and again at 2-8 °C for 9 days. During the last storage period crystallization was observed and the obtained crystals were collected, air dried and investigated by PXRD, which confirmed the receipt of crystalline acalabrutinib *L*-malate.

### Preparation of acalabrutinib L-malate

Acalabrutinib (500 mg, e.g. prepared according to Examples 6 of WO 2013/010868 A1) was dissolved in ethanol (10 mL) upon heating to 50 °C. To the solution *L*-malic acid (140 mg) was added. The mixture was then seeded with the acalabrutinib *L*-malate seed crystals obtained above. The mixture was allowed to cool to room temperature and stirred for 16 hours. The obtained crystals were collected by filtration and dried at room temperature under vacuum (5-10 mbar) for 5 hours to obtain crystalline acalabrutinib *L*-malate (520 mg, yield: 81% of theory, seeds not considered).

### Example 4: Characterization of acalabrutinib L-malate

### Powder X-ray diffraction

PXRD was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta. Thus, the diffraction peak of crystalline acalabrutinib *L*-malate of the present invention that appears for example at 6.2° 2-Theta can appear in the range of from 6.0 to 6.4° 2-Theta, preferably in the range of from 6.1 to 6.3° 2-Theta on most X-ray diffractometers under standard conditions.

A representative diffractogram of crystalline acalabrutinib *L*-malate of the present invention is displayed in Figure 5 herein. The corresponding reflection postitions in the range of 2 to 30 ° 2-Theta are provided in Table 5 below.

**Table 5: PXRD reflections of crystalline acalabrutinib L-malate of the present invention is in the range of from 2 to 30° 2-Theta; A typical precision of the 2-Theta values is in the range of ± 0.2° 2-Theta, preferably of ± 0.1° 2-Theta.**

| **Reflection position** **[° 2-Theta]** | **Reflection position** **[° 2-Theta]** | **Reflection position** **[° 2-Theta]** | **Reflection position** **[° 2-Theta]** |
|---|---|---|---|
| 4.2 | 14.2 | 20.7 | 25.5 |
| 6.2 | 15.6 | 21.2 | 25.9 |
| 8.4 | 16.9 | 22.1 | 26.3 |
| 8.9 | 17.9 | 22.9 | 26.9 |
| 11.9 | 18.7 | 23.8 | 27.6 |
| 12.4 | 19.6 | 24.4 | 29.4 |
| 12.9 | 20.2 | 24.9 | |

### Fourier transform infrared spectroscopy

The FTIR spectrum of acalabrutinib *L*-malate was recorded (obtained) on an MKII Golden Gate™ Single Reflection Diamond ATR cell with a Bruker Tensor 27 FTIR spectrometer with 4 cm⁻¹ resolution at a temperature in the range of from 20 to 30 °C. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of ± 2 cm⁻¹. Thus, the infrared peak of crystalline acalabrutinib *L*-malate that appears for example at 3471 cm⁻¹ can appear between 3469 and 3473 cm⁻¹ on most infrared spectrometers under standard conditions.

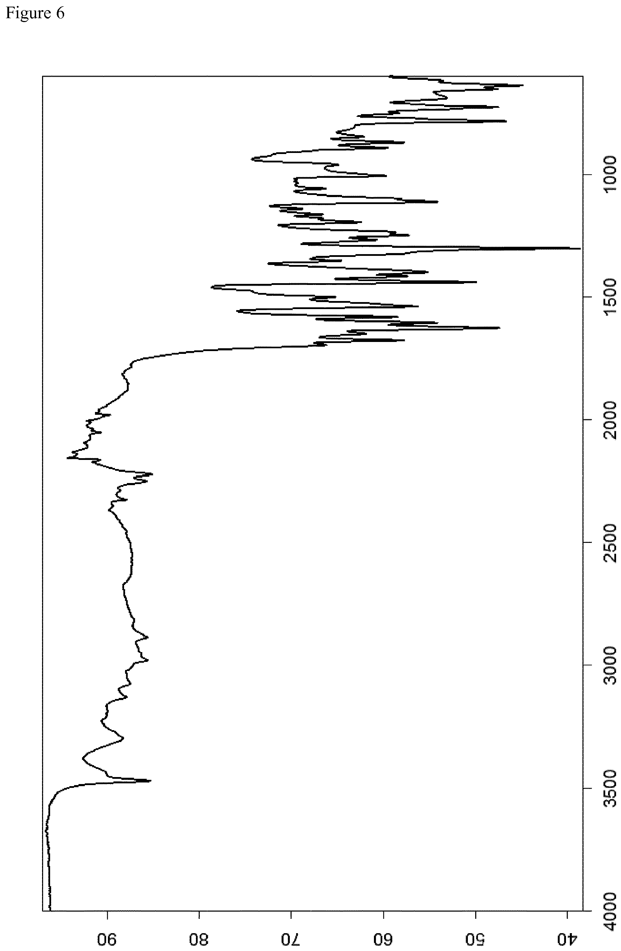

A representative FTIR spectrum of acalabrutinib *L*-malate according to the present invention is displayed in Figure 6 and the corresponding peak list is provided in Table 6 below.

**Table 6: FTIR peak list of crystalline acalabrutinib L-malate according to the present invention; a typical precision of the wavenumbers is in the range of ± 2 cm⁻¹.**

| **Wavenumber** **[cm⁻¹]** | **Wavenumber** **[cm⁻¹]** | **Wavenumber** **[cm⁻¹]** | **Wavenumber** **[cm⁻¹]** |
|---|---|---|---|
| 3471 | 1605 | 1267 | 894 |
| 2979 | 1581 | 1249 | 871 |
| 2885 | 1539 | 1196 | 848 |
| 2250 | 1501 | 1164 | 784 |
| 2223 | 1440 | 1141 | 751 |
| 1696 | 1416 | 1112 | 726 |
| 1677 | 1399 | 1059 | 690 |
| 1650 | 1353 | 1006 | 652 |
| 1626 | 1302 | 961 | 638 |

### Differential scanning calorimetry

DSC was performed on a Mettler Polymer DSC R instrument. Acalabrutinib *L*-malate (3.75 mg) was heated in a 40 microliter aluminium pan with a pierced aluminium lid from 25 to 210 °C at a rate of 10 K/min. Nitrogen (purge rate 50 mL/min) was used as purge gas.

The DSC curve of acalabrutinib *L*-malate shows an endothermic melting peak with an onset temperature of about 182 °C and a peak maximum temperature of about 184 °C (see also Figure 7 herein).

### Thermogravimetric analysis

TGA was performed on a Mettler TGA/DSC 1 instrument. Acalabrutinib *L*-malate (10.28 mg) was heated in a 100 microliter aluminum pan closed with an aluminum lid. The lid was automatically pierced at the beginning of the measurement. The sample was heated from 25 to 220 °C at a rate of 10 K/min. Nitrogen (purge rate 50 mL/min) was used as purge gas.

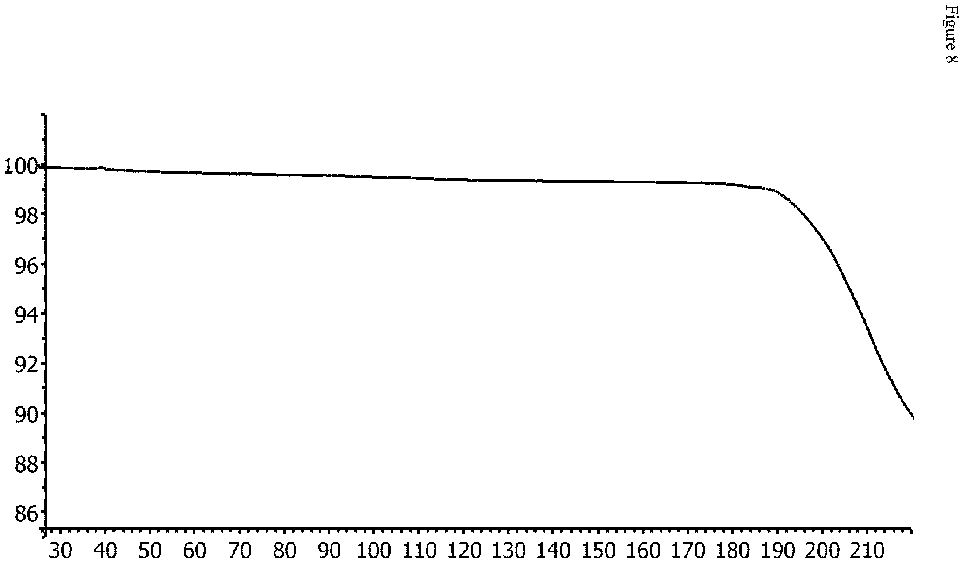

The TGA curve of acalabrutinib *L*-malate shows a mass loss of about 0.8 weight% up to a temperature of about 190 °C (see also Figure 8 herein). Hence, it can be concluded that neither water nor organic solvents are part of the crystal structure but the mass loss may rather be due to the release of residual water and/or solvent, which is loosely bound on the surface.

### Gravimetric moisture sorption

A moisture sorption isotherm of crystalline acalbrutinib *L*-malate was recorded along with other samples with an SPSx-1µ moisture sorption analyzer (ProUmid, Ulm). In the sorption cycle the relative humidity was increased from 0% to approximately 90% in 5% steps. The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C.

During the sorption cycle crystalline acalabrutinib *L*-malate of the present invention showed a mass increase (water uptake) of about 1.2 weight% from 0 to 80% RH and of about 1.6 weight% from 0 to 90% RH.

## Claims

1. Crystalline acalabrutinib *L*-pyroglutamate.

2. The crystalline acalabrutinib *L*-pyroglutamate of claim 1 **characterized by** the chemical structure according to formula (IIa) 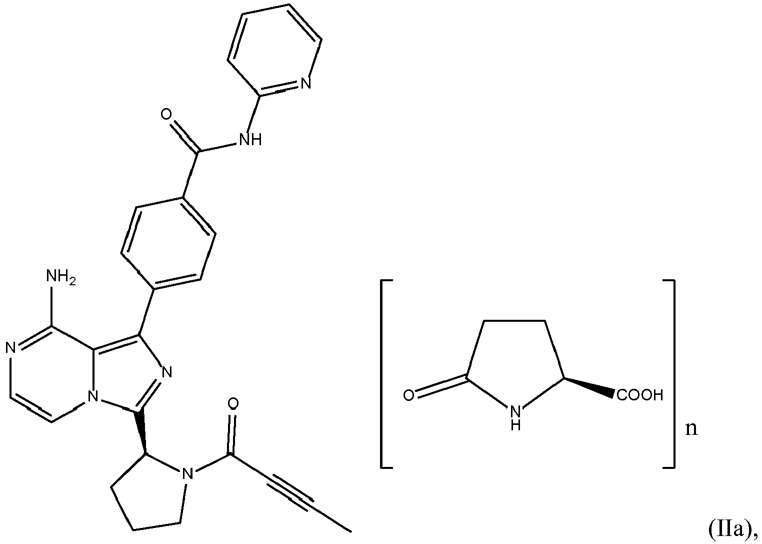 wherein n is in the range of from 0.8 to 1.2.

3. The crystalline acalabrutinib *L*-pyroglutamate according to claim 1 or 2, **characterized in** being an anhydrous and non-solvated form.

4. The crystalline acalabrutinib *L*-pyroglutamate according to any one of the preceding claims **characterized by** having a powder X-ray diffractogram comprising reflections at 2-Theta angles of (6.6 ± 0.2)°, (19.4 ± 0.2)° and (22.2 ± 0.2)°, when measured at a temperature in the range of from 20 to 30 °C with Cu-Kalpha_{1,2} radiation having a wavelength of 0.15419 nm.

5. The crystalline acalabrutinib *L*-pyroglutamate of claim 4 **characterized by** having a powder X-ray diffractogram comprising additional reflections at 2-Theta angles of (10.5 ± 0.2)° and/or (12.4 ± 0.2)°, when measured at a temperature in the range of from 20 to 30 °C with Cu-Kalpha_{1,2} radiation having a wavelength of 0.15419 nm.

6. The crystalline acalabrutinbib *L*-pyroglutamate according to any one of the preceding claims **characterized by** having a Fourier transform infrared spectrum comprising peaks at wavenumbers of (3318 ± 2) cm⁻¹, (2225 ± 2) cm⁻¹ and (1625 ± 2), when measured at a temperature in the range of from 20 to 30 °C with a diamond attenuated total reflection cell.

7. Crystalline acalabrutinib *L*-malate.

8. The crystalline acalabrutinib *L*-malate of claim 7 **characterized by** the chemical structure according to formula (IIb) 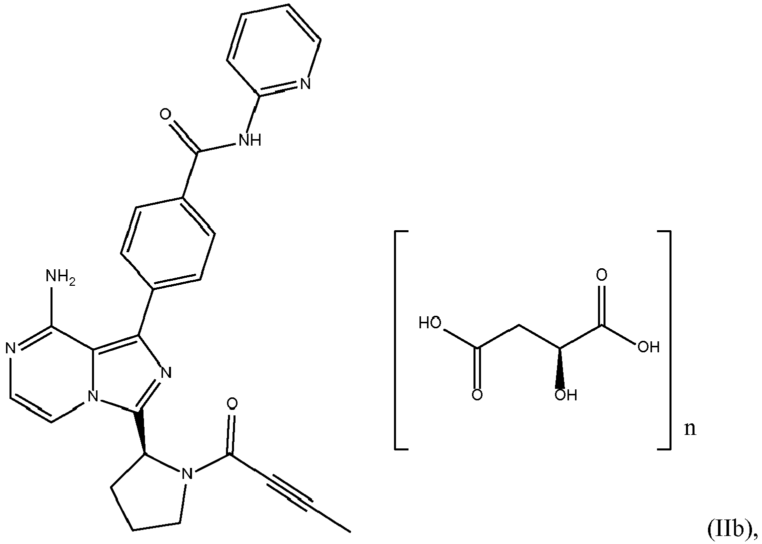 wherein n is in the range of from 0.8 to 1.2.

9. The crystalline acalabrutinib *L*-malate according to claim 7 or 8, **characterized in** being an anhydrous and non-solvated form.

10. The crystalline acalabrutinib *L*-malate according to any one of claims 7 to 9 **characterized by** having a powder X-ray diffractogram comprising reflections at 2-Theta angles of (6.2 ± 0.2)°, (11.9 ± 0.2)° and (12.4 ± 0.2)°, when measured at a temperature in the range of from 20 to 30 °C with Cu-Kalpha_{1,2} radiation having a wavelength of 0.15419 nm.

11. The crystalline acalabrutinib *L*-malate of claim 10 **characterized by** having a powder X-ray diffractogram comprising additional reflections at 2-Theta angles (8.9 ± 0.2)° and/or (22.9 ± 0.2)°, when measured at a temperature in the range of from 20 to 30 °C with Cu-Kalpha_{1,2} radiation having a wavelength of 0.15419 nm.

12. The crystalline acalabrutinbib *L*-malate according to any one of claims 7 to 11 **characterized by** having a Fourier transform infrared spectrum comprising peaks at wavenumbers of (3471 ± 2) cm⁻¹, (2223 ± 2) cm⁻¹ and (1626 ± 2), when measured at a temperature in the range of from 20 to 30 °C with a diamond attenuated total reflection cell.

13. A pharmaceutical composition comprising the crystalline acalabrutinib *L-*pyroglutamate as defined in any one of claims 1 to 6 or the crystalline acalabrutinib *L-*malate as defined in any one of claims 7 to 12, and at least one pharmaceutically acceptable excipient.

14. The pharmaceutical composition of claim 13 for use in the treatment of cancer, in particular mantle cell lymphoma and chronic lymphocytic leukemia.
